# EUROPEAN PATENT SPECIFICATION

(11) **EP 2 793 625 B1**
(45) Date of publication and mention of the grant of the patent: **27.09.2017**
(21) Application number: 12809320.0
(22) Date of filing: 20.12.2012
(51) Int. Cl.: A24D 3/04

(54) **SMOKING ARTICLES, AND OTHER FLOW DELIVERY ARTICLES**
RAUCHARTIKEL UND ANDERE FLUSSFREISETZUNGSARTIKEL
ARTICLES À FUMER, ET AUTRES ARTICLES ÉMETTEURS DE FLUX

(30) Priority: 20.12.2011 GB 201121920; 20.12.2011 GB 201121922
(43) Date of publication of application: 29.10.2014
(60) Divisional application: 17186873.0
(73) Proprietor: British American Tobacco (Investments) Limited, London WC2R 3LA (GB)
(72) Inventor: JOHNSON, Trevor, Chippenham Wiltshire SN14 6XL (GB); FROBISHER, Paul, Clevedon BS21 6UW (GB); AWTY, Edward, London WC2R 3LA (GB); NICHOLLS, Jane, London WC2R 3LA (GB); NANDRA, Charanjit, London WC2R 3LA (GB); NEWNHAM, Michael, Clevedon BS21 6UW (GB); BOAST, David, Chippenham Wiltshire SN14 8DL (GB); SMITH, Simon, Cambridge CB4 0DW (GB); ABERCROMBIE, Stuart, Cambridge CB4 0DW (GB)
(74) Representative: EIP
(86) International application number: PCT/GB2012/053200
(87) International publication number: WO 2013/093469

(56) References cited:
- WO-A1-96/24407
- WO-A1-2011/051115
- WO-A2-2006/129304
- GB-A- 1 118 341
- GB-A- 1 404 338
- US-A- 4 253 508
- US-A- 4 867 182
- US-A1- 2008 276 948
- US-A1- 2009 283 103
- US-A1- 2011 265 806

## Description

### Field

This disclosure relates to a flow delivery article. In particular, but not exclusively, it relates to a flow delivery article having a vibration component to provide tactile stimulation to a user.

### Background

Known cigarettes deliver smoke in a continuous stream in proportion to the drawing effort provided by the smoker. Menthol cigarettes are available, which provide a stream of smoke which is flavoured with menthol.

### Description

This disclosure provides a flow delivery article such as a smoking article. Flow delivery articles deliver a gaseous flow to the mouth of a user. In various embodiments, the flow delivery article comprises a vibration component to provide tactile stimulation to a user.

As used herein the term "flow delivery article" includes products which deliver flow such as smoking articles, heat-not-burn products, electronic-cigarettes, and aerosol/mist/vapour delivery articles. The flow delivery article may include a tobacco industry article such as a cigarette or e-cigarette.

The flow provided by the flow delivery article comprises a gaseous flow. The flow delivery article may deliver flow in the form of smoke, aerosol, air, vapour, mist or a mixture thereof.

In an embodiment the flow delivery article comprises a flow pathway and a flow-driven vibration component, wherein the vibration component is configured to provide vibration driven by flow passing along the flow pathway.

In an embodiment the flow delivery article is a smoking article comprising a vibration component, wherein the vibration component comprises a movable member which is arranged to move in response to receiving smoke flow.

In an embodiment the flow delivery article comprises a source of inhalable agent and a vibration component, wherein the vibration component is longitudinally adjacent to or longitudinally spaced from the source of inhalable agent. The source of the inhalable agent may be tobacco (for example in the form of a tobacco rod), which upon combustion provides an inhalable agent in the form of tobacco smoke. Alternatively, the source of the inhalable agent may be a reservoir comprising an inhalable gas or liquid.

In embodiments, the vibration component is located at the mouth end of the flow delivery article.

In some embodiments, the flow delivery article is a smoking article having a filter, wherein the filter is arranged between the vibration component and the source of inhalable agent. In other embodiments, the vibration component is arranged within the filter. In yet further embodiments, the vibration component is arranged between the filter and the source of the inhalable agent.

The vibration component may be configured to generate vibration in response to receiving flow drawn by the user. The generated vibration may increase when the flow drawn by the user increases and decrease when the flow drawn by the user decreases.

The vibration component may comprise a moveable member which is configured to move continually when receiving smoke flow.

The flow delivery article may have a peripheral region to contact the lips of the user. The vibration component may be arranged to vibrate said peripheral region to provide tactile stimulation to the user's lips.

The flow delivery article may have a peripheral region to contact the fingers of the user. The vibration component may be arranged to vibrate said peripheral region to provide tactile stimulation to the user's fingers.

In embodiments, the vibration component comprises an eccentrically-weighted rotary member and/or an eccentrically mounted rotary member.

The vibration component may comprise a contact surface and a rotary member to repeatedly contact said contact surface to cause vibration. The vibration component may include a resilient flap comprising said contact surface.

The flow delivery article may comprise a movable member arranged to move in response to receiving flow. The movable member may comprise a rotary member arranged to rotate in response to receiving flow.

The vibration component may comprise a flow conduit which is arranged to preferentially direct flow towards a peripheral surface of the rotary member.

The rotary member may comprise a turbine. The turbine may comprise a substantially spherical turbine, a fan turbine, a cylindrical turbine, a positive displacement turbine, an axial turbine, or a progressive cavity turbine. The flow delivery article may include a flow conduit to preferentially direct flow towards a region of the turbine, e.g: towards one of the hemispheres of a spherical turbine.

In embodiments, the progressive cavity turbine may comprise a rotor which is circular in cross-section and a stator housing which comprises a double lobed helical lumen.

In some embodiments, the movable member comprises an aerodynamic element arranged to move in response to receiving flow.

The vibration component may comprise a smoke pathway, and the vibration component may be configured to vibrate in response to receiving flow drawn through the smoke pathway. The pathway may include a venturi section.

The vibration component may comprise a body and a movable member to repeatedly contact one or more regions of the body to cause vibration. The vibration component may further comprise a coupling member to couple the movable member to the body. The coupling member may be formed from a resilient material. In embodiments, the coupling member may be integral with the movable member.

In embodiments, the movable member is adapted to move without a predefined pattern.

The movable member may comprise a plurality of aerofoils. In addition, or as an alternative, the movable member may comprise a plurality of bluff bodies. The movable member may comprise at least two different surface regions having different aerodynamic properties.

In some embodiments, the aerodynamic element is adapted to flutter in the flow drawn by the user.

The vibration component may comprise one or more air conduits to allow outside air to mix with the smokestream.

The flow delivery article may be a smoking article. The smoking article may comprise a tobacco rod component and a filter rod component.

The flow delivery article may comprise a motor configured to drive vibration of the vibration component.

This disclosure also provides a filter for a smoking article comprising a vibration component to provide tactile stimulation to a user.

In embodiments, the amplitude of vibration may vary or may be constant. For example, the amplitude may increase when the flow drawn by the user increases and decrease when the flow drawn by the user decreases.

In some embodiments the vibration component vibrates at a constant frequency. In other embodiments, the frequency of vibration may vary. For example, the frequency may increase when the flow drawn by the user increases and decrease when the flow drawn by the user decreases.

As used herein, the term "smoking article" includes smokeable products such as cigarettes, cigars, cigarillos and pipes, whether based on tobacco, tobacco derivatives, expanded tobacco, reconstituted tobacco or tobacco substitutes and also heat-not-burn products.

A smoking article may include a combustion-based smoking article such as a cigarette. Alternatively, a smoking article may include a non-combustion-based article such as an electronic cigarette, or other non-combustion-based component which is smoked in use.

In order that the invention(s) of this disclosure may be more fully understood, embodiments thereof will now be described by way of example only, with reference to the accompanying drawings, in which:
Figure 1a is a view showing an axial section through a cigarette, and illustrates a vibration component positioned between a tobacco rod and a filter plug;
Figure 1b is a sectional view showing the vibration component in more detail;
Figure 1c is an end view of the vibration component;
Figure 1d is a perspective view of the fan and shaft of the vibration component;
Figure 2 illustrates another vibration component;
Figure 3a is a sectional view of another vibration component;
Figure 3b is a sectional view of yet another vibration component;
Figure 4a is a perspective view of a cigarette comprising a vibration component positioned at the mouth end, adjacent to a filter plug;
Figure 4b is a perspective view of the rotatable screw element of the vibration component of Figure 4a;
Figure 5a is a perspective view of a cigarette comprising a vibration component positioned at the mouth end, adjacent to a filter plug;
Figure 5b shows part of the vibration component;
Figure 5c is a perspective view of the helical rotor of the vibration component;
Figure 5d shows the mouth end part of the vibration component;
Figure 6a is a sectional view yet another vibration component;
Figure 6b is a perspective view of the vibration component of Figure 6a, with part of the body removed;
Figure 6c illustrates the exterior of the body of the vibration component of Figure 6a;
Figure 7a is a sectional view of yet another vibration component;
Figure 7b is an end view of the vibration component of Figure 7a;
Figure 8 illustrates a variation of the vibration component of Figure 7a;
Figure 9a is a sectional view of yet another vibration component;
Figure 9b is a perspective view of the vibration component of Figure 9a, with part of the body removed;
Figure 9c illustrates the exterior of the vibration component of Figure 9a;
Figure 10 is a sectional view of yet another vibration component;
Figure 11a is a perspective view of yet another vibration component, with part of the body removed;
Figure 11b shows the movable member of the vibration component of Figure 11a;
Figure 12 is a perspective view of yet another vibration component, with part of the body removed;
Figure 13a is a perspective view of a cigarette comprising a vibration component positioned between a tobacco rod and a filter plug;
Figure 13b is a perspective view showing the vibration component in more detail, with part of the body removed;
Figure 14 illustrates a variation of the vibration component of Figure 13a and b, with part of the body removed:

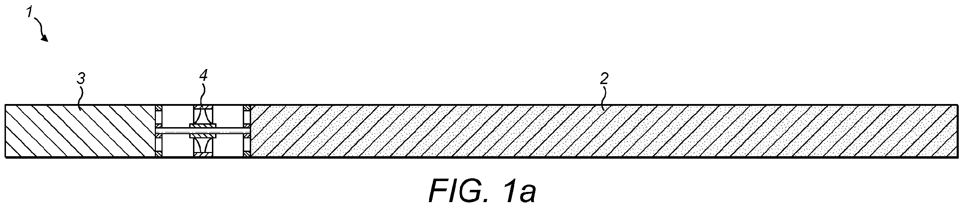

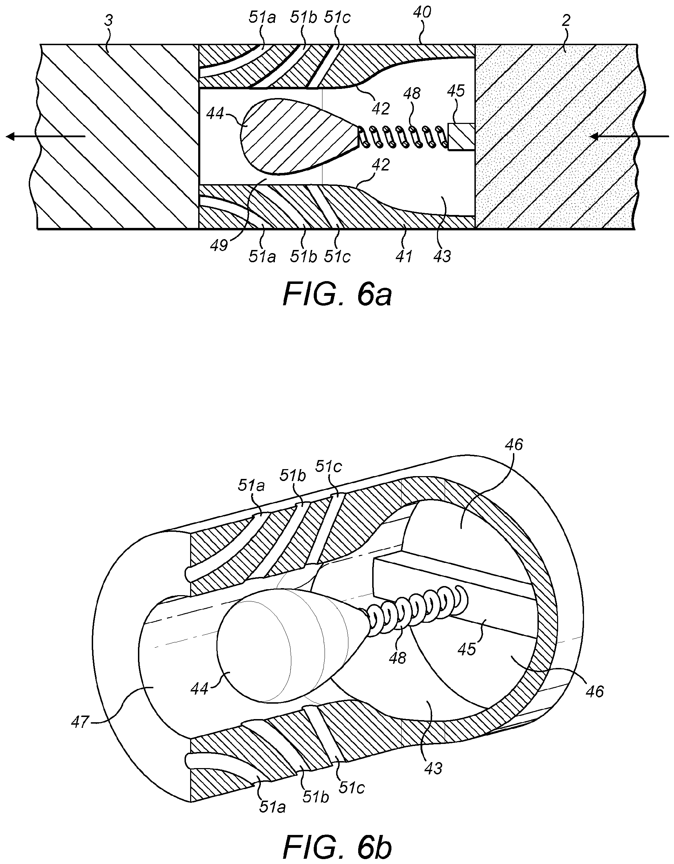

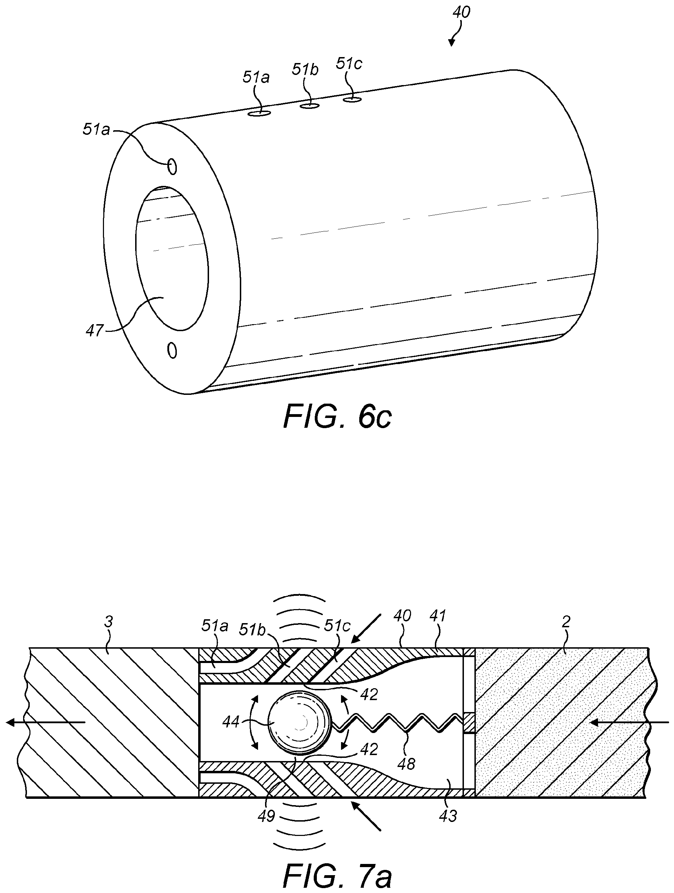

Figures 6a to 14 disclose embodiments which do not form part of the claimed invention. Figure 1a illustrates a flow delivery article in the form of a smoking article 1. As shown in Figure 1a, smoking article 1 comprises a tobacco rod component 2, a filter plug component 3, and a vibration component 4, which is positioned between the tobacco rod 2 and the filter plug 3. The tobacco rod 2, filter plug 3, and vibration component 4 are longitudinally aligned and wrapped with a tipping paper (not shown) to hold them together.

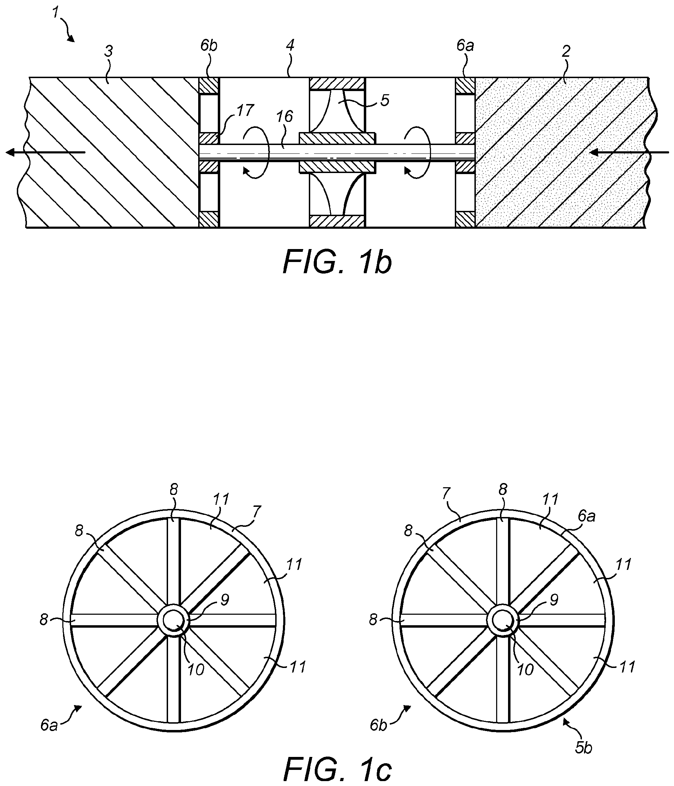

Referring to Figure 1b, which shows the vibration component 4 in more detail, component 4 comprises a rotary fan 5 which is driven by the smoke flow drawn from the tobacco rod 2. The fan 5 is eccentrically weighted and so causes vibration when it rotates. In this way, vibration component 4 generates vibration.

Turning to a more detailed description of component 4, as shown in Figure 1b, component 4 has a body having circular end regions 6a, 6b, which are fixed in position relative to the rod 2 and filter plug 3. As shown in Figure 1c, the end regions 6a, 6b have a cartwheel configuration comprising an outer ring 7 supported by radially-extending spokes 8. The spokes 8 extend from hub 9, which defines a circular opening 10. As shown, the spaces between the spokes 8 define openings 11 to permit the passage of smoke drawn from the tobacco rod 2. The spokes 8 may have any suitable shape. In some embodiments one or more of the spokes 8 is shaped as a stationary aerofoil (e.g: in the form of a stationary vane or blade) to control the flow passing through the vibration component to improve energy absorption by the rotary fan 5.

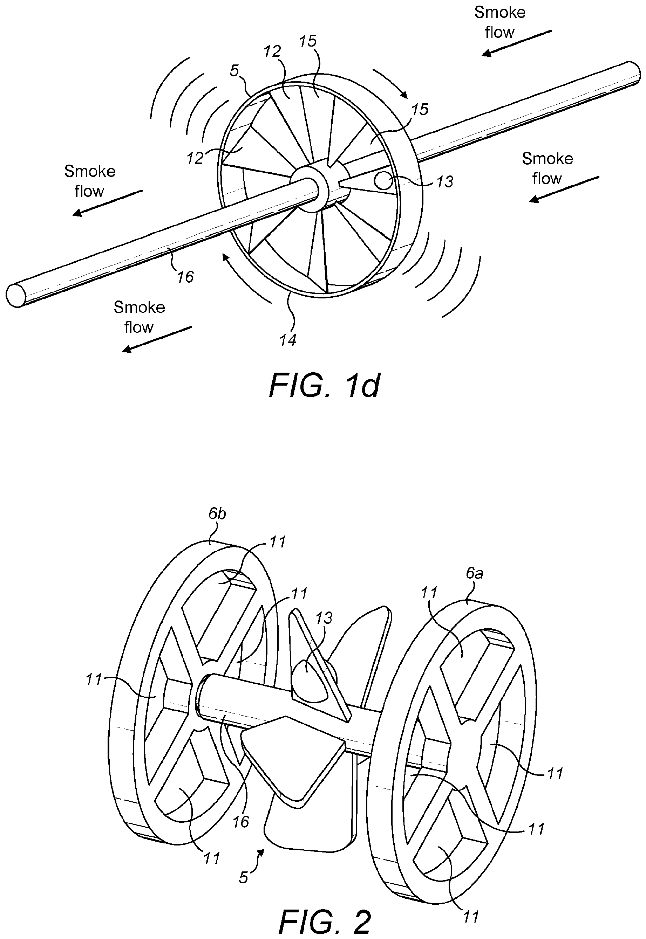

Figure 1d shows a perspective view of the fan 5. As shown, fan 5 comprises a plurality of vanes 12, one of which has a weighted and unbalancing mass 13 attached to it, so that the fan is eccentrically-weighted. In other words, the centre of mass of the fan 5 does not coincide with its geometric centre. The vanes 12 may be supported by an outer ring 14 and define openings 15 to permit the passage of smoke.

The fan 5 is fixed to and rotates with a shaft 16, which is rotatably mounted in the circular openings 8 of the end regions 6a, 6b by way of bearings 17. In this way, fan 5 and shaft 16 are free to rotate relative to the stationary body 6a, 6b.

In use, a smoker draws smoke from tobacco rod 2, which passes through the openings 11 in the end region 6a and impacts the eccentrically-weighted fan 5, causing it and the eccentrically positioned mass 13 to rotate. The smoke then passes through the openings 15 in the fan 5, through the openings 7 in the end region 6b, through the filter plug 3 and into the smoker's mouth.

Rotation of the eccentrically-weighted fan 5 causes the component 4 to vibrate. This vibration is imparted to the filter plug 3 and also to tobacco rod 2, and is perceived tactually by the smoker. In particular, the smoker feels the vibratory movement of the peripheral region of the filter plug 3 which is contacting his or her lips, and also feels the vibratory movement of the peripheral region of the tobacco rod 1 which is contacting his or her fingers. Fan 5 rotates faster if the smoke flux is higher, such that vibration increases and decre ses according to the amount of draw applied to the smoking article 1.

Figure 2 shows a variation of the vibration component 4 of Figure 1a, and the same reference numerals are used for corresponding features. As shown, in the example of Figure 2a, the outer supporting ring 14 of the fan 5 is omitted, and the cartwheel configuration of the smoke openings 11 through the end regions 6a, 6b is different to the configuration of Figure 1c. Again, the spokes may have any suitable shape and in some embodiments may comprise one or more stationary aerofoils.

Many further variations and modifications of the vibration component 4 are possible. In some embodiments, the fan 5 may be unbalanced by means of unevenly spaced vanes. Furthermore, instead of, or in addition to being unbalanced, such as by means of an unbalancing mass 13, fan 5 may include one or more axially protruding members (not shown) positioned on one or more of the rotary vanes 12 and/or the rotary outer ring 14. As the fan rotates, the protruding member(s) may repeatedly strike a resilient flap, thereby causing vibration. The resilient flap may be attached to the stator body, or to the tipping paper wrapper of the smoking article 1.

Further, although the fan is shown in Figure 1b rotatably mounted to both end regions 6a, 6b, the fan 5 may alternatively be rotatably mounted to a single end region 6a. In this case, the fan may rotate closely adjacent to the fixed end region 6a.

Still further, although the shaft 16 is described above as rotating relative to the end regions 6a, 6b of the vibration component 4, alternatively, the shaft 16 may be fixed relative to the end regions 6a, 6b. In this case, the fan 6 may include a through-hole for the shaft, and may be rotatably mounted on the fixed shaft with a bearing.

In yet further embodiments the vibration component 4 may comprise a plurality of fans 5, such as, for example two, three, four, or five fans in sequence. The fans 5 may be fixed to a single shaft 16, or alternatively, the fans 5 may be arranged to be capable of rotating independently. The fans 5 may be separated by one or a number of stators, each comprising a stationary aerofoil to control the flow passing through the vibration component to improve energy absorption by the fans.

In one embodiment, each fan comprises a plurality of vanes and each stator comprises a plurality of vanes. The orientation of the vanes of the fans may be different to (e.g: opposite to) the vanes of the fans.

The use of a plurality of fans may reduce the lag time between the smoker commencing the draw and perceiving the resulting vibration.

In embodiments, in addition to, or as an alternative to the smoke flow drawn by the user, the fan 5 may be driven by drawn flow of air and/or flavourant.

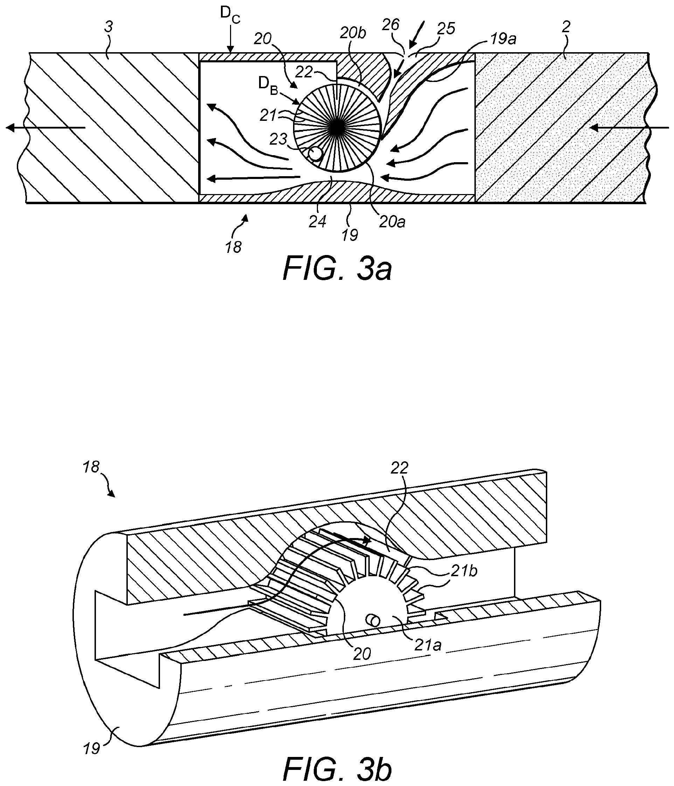

Figure 3a illustrates another vibration component 18 in place between a tobacco rod 2 and a filter plug 3. As shown, component 18 comprises a stator body 19 defining a smoke conduit containing a spherically-shaped turbine 20, which is driven to rotate by the smoke flow drawn from tobacco rod 2.

As shown in Figure 3a, the inner surface 19a of body 19 is shaped to direct smoke drawn from the tobacco rod 2 towards the lower hemisphere 20a of ball turbine 20. The ball 20 is formed of a plurality of vanes 21. The vanes extend outwardly from the center of the ball 20 at different angles of inclination, so that neighbouring vanes are angularly separated from one another. Each vane has a semi-circular cross section. The vanes 21 receive flow from the tobacco rod so as to cause the turbine 20 to rotate. By means of the shaping of the inner surface 19a of body 19, smoke is substantially directed towards the peripheral surface of the ball turbine 20 to increase the torque applied to the turbine 20. The ball 20 is rotatably mounted in a cavity inside body 19 on an axle (not shown), which passes through the ball.

In the embodiment shown, the body 19 includes a protrusive resilient flap 22, which extends between the vanes 21 and is repeatedly struck by the vanes 21 as the ball 20 rotates. This causes vibration of the component 18, which is imparted to the tobacco rod 2 and filter 3 and is perceived tactually by the smoker. The ball rotates faster when the smoke flux is higher, such that the vibration increases and decreases according to the amount of draw on the smoking article 1. The flapper 22 may also serve to baffle/prevent smoke re-circulation away from the mouth end of the filter 3.

The axle may pass through the centre of the ball 20, or alternatively may pass away from the centre so that the ball rotates eccentrically, so as to provide an additional or alternative means of vibration. Alternatively, or in addition, the ball 20 may be eccentrically weighted by way of an unbalancing mass 23 located near the periphery of the ball 20.

As illustrated in Figure 3a, smoke passes through a constricted region 24 as it flows past the ball 20. This leads to a venturi effect which increases the velocity of the smoke flow.

A secondary channel 25 having an inlet 26 at the periphery of smoking article 1 is also provided to permit diluting air to be drawn in and mixed with the smoke flow. In some embodiments, some or all of the diluting air may be drawn via a chamber comprising a flavourant. As shown, the smoke from tobacco rod 2 and air from channel 25 are routed to opposite hemispheres 20a, 20b of ball 20.

In some embodiments, there may be a gap between the ball 20 and the flow conduit. The diameter of the ball 20 may be between 0.4Dc and 0.9 Dc, where Dc is the diameter of the flow conduit containing the ball 20. In some examples, there may be no substantial gap between the ball 20 and the conduit.

In some embodiments, the vanes 21 do not protrude from the centre of the ball 20, but rather the ball 20 comprises a solid sphere. The vanes 21 are provided by a plurality of ridges on the surface of the sphere, with neighbouring ridges angularly separated from one another.

Figure 3b illustrates a variation of the vibration component 18 of Figure 3a, and the same reference numerals are retained for corresponding features. As shown, in the example of Figure 3b, the vibration component 18 comprises a stator body 19 defining a smoke conduit containing a movable member 20 in the form of a cylindrical turbine or paddlewheel. The paddlewheel 20 is driven to rotate by the smoke flow drawn from tobacco rod 2.

The paddlewheel 20 comprises a cylindrical body 21a and a plurality of longitudinal paddles 21b. The paddles 21b extend outwardly from the circumferential surface of the cylindrical body 21a at different angles of inclination, so that neighbouring paddles are angularly separated from one another. The paddlewheel 20 is rotatably mounted in a cavity inside body 19. In use, the paddles 21b receive flow from the tobacco rod so as to cause the paddlewheel 20 to rotate.

The inner surface 19a of body 19 is shaped to direct smoke drawn from the tobacco rod 2 substantially towards and around the peripheral surface of the paddlewheel 20. There may be a gap between the body 19 and the paddlewheel 20, which may allow smoke to be drawn around the paddlewheel 20.

In the embodiment shown, the body 19 includes a protrusive resilient flap 22, which extends between the paddles 21b and is repeatedly struck by the paddles 21b as the paddlewheel 20 rotates, causing vibration of the component 18.

The paddlewheel rotates faster when the smoke flux is higher, such that the vibration increases and decreases according to the amount of draw on the smoking article 1. The flapper 22 may also serve to baffle/prevent smoke re-circulation away from the mouth end of the filter 3.

As an alternative or in addition to the flapper 22, the paddlewheel 20 may be eccentrically weighted to cause vibration when it rotates. Furthermore, the axis about which the paddlewheel 20 rotates may be off-centred to cause eccentric rotation. In other words, the paddlewheel 20 may be arranged so that the centre of mass of the paddlewheel 20 does not lie on the axis of rotation.

In the embodiment shown in Figure 3b, the axis of rotation of the paddlewheel 20 is aligned perpendicularly to the longitudinal axis of the smoking article 1. In other embodiments, however, the paddlewheel 20 is aligned at an oblique angle to the longitudinal axis of the smoking article 1. In these embodiments, to increase the torque applied by the smoke flow to the paddlewheel 20, the paddles 21b may have a helical configuration around the circumferential surface of the cylinder 21a.

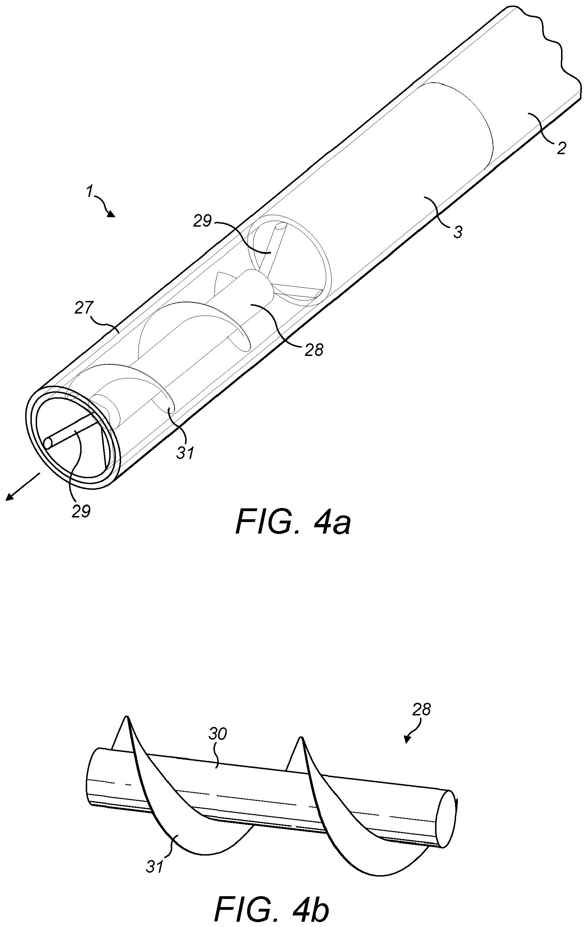

Figure 4a illustrates another vibration component 27 in place at the mouth end of a flow delivery article, which in this case is a smoking article 1. The vibration component 27 has the form of an axial turbine. As shown, the vibration component 27 comprises a rotatable screw element 28 which is driven to rotate by the gaseous flow drawn from tobacco rod 2 through filter plug 3. The screw element is rotatably supported by stationary support elements 29 located at each end of the screw element 28. In other embodiments, the screw element may be rotatably supported at one end only, for example, by means of a cantilevered central shaft.

In the embodiment shown, the screw element 28 is eccentrically weighted and so causes vibration when it rotates.

Figure 4b shows the screw element 28 in more detail. The screw element 28 comprises a rod 30 with one or more helical vanes 31 spiralling along the length of the rod 30. The vanes 31 receive flow drawn from the tobacco rod 2 through the filter plug 3 so as to cause the screw element 28 to rotate.

The vibration components of Figure 4 are positive displacement devices. This means that substantially no flow is drawn through the device in the absence of rotation of the device. As a result, the lag time between the smoker commencing the draw and perceiving the resulting vibration is minimised.

Furthermore, the rate of rotation of the screw element 28 is generally proportional to the strength of draw applied to the smoking article 1. Thus the screw element 28 rotates faster when the smoke flux is higher, and the degree of vibration increases and decreases in response to the amount of draw on the smoking article 1.

Many methods of eccentrically weighting the screw element 28 are possible. For example, the screw element 28 may be eccentrically weighted by means of an unbalancing mass incorporated, for example, within the vane. In addition, or as an alternative, the axis about which the screw element 28 rotates may be off-centred so that the element rotates eccentrically. In general, the screw element 28 is arranged so that the centre of mass of the screw element 28 does not lie on the axis of rotation.

Many variations and modifications of the vibration component 27 are possible. For example, instead of a central rod 30, the screw element 28 may comprise a hollow cylinder which is arranged to rotate about a fixed central shaft.

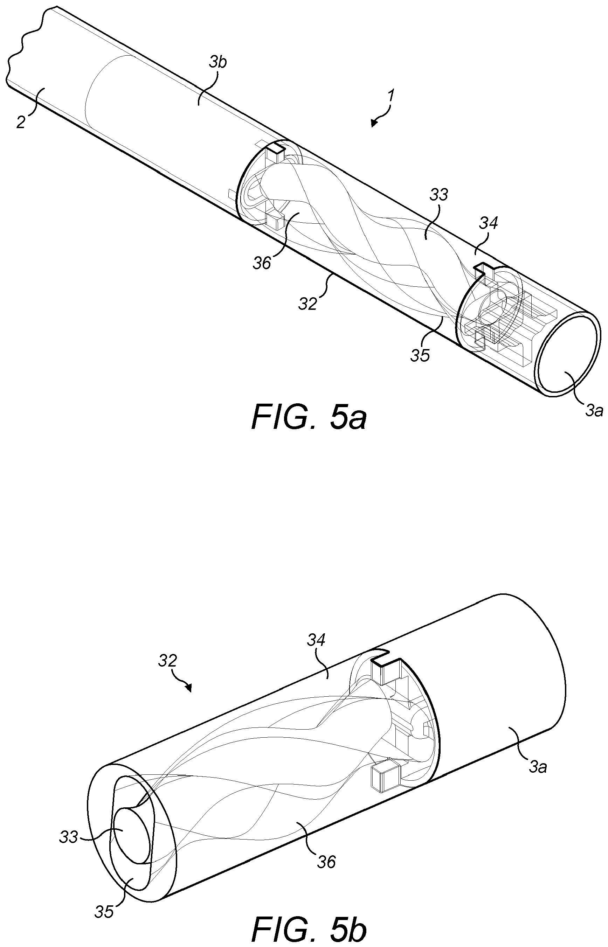

Figure 5a illustrates another vibration component in place between a mouthpiece 3a and a filter plug section 3b at the mouth end of a smoking article 1. The vibration component 32 comprises a progressive cavity device 32.

Progressive cavity devices (such as the eccentric screw pump, also known as a cavity pump or Moineau pump) are well known *per se* and will not be described in detail here. Briefly, a progressive cavity device comprises a rotary element configured to rotate to cause one or more cavities to move through the device, thereby to transfer flow.

It is known *per se* to provide a progressive cavity device as a pump device to transfer fluid or as a motor, for example in oilfield applications. In contrast, the progressive cavity device 32 of Figure 5a is a turbine driven by the flow drawn from the smoking article 1. It has been found that the device 32 generates vibration when it rotates.

Turning to a more detailed description of the device 32, as shown in Figure 5a, the device 32 comprises a helical rotor 33 and a stator housing 34. The stator housing 34 has an inner surface 35 defining a smoke conduit.

As shown in Figure 5b, the rotor 33 of the vibration component 32 is circular in cross-section. The inner surface 35 of the stator housing 34 is shaped along its length to form a double lobed helix, and a number of fixed size cavities 36 are thus formed within the housing 34 between the rotor 33 and the internal surface 35 of the housing at any particular rotational position of the rotor.

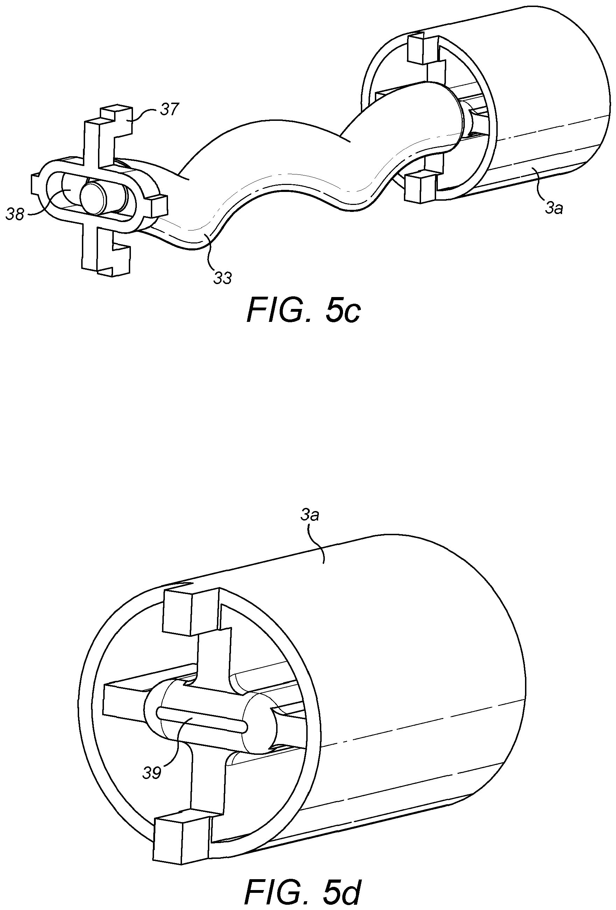

As shown in Figure 5c, the vibration component 32 further comprises a support element 37 which is fixed in position relative to the housing 34 and to the filter plug section 3b. As shown, the rotor 33 is rotatably mounted to the support element 37 by means of an elongated slot 38 formed in the support element 37. In use, as the rotor 33 rotates, the end of the rotor moves back and forth along the elongated slot 38. The end of the rotor 33 may comprise a bearing to improve rotation. The other end of the rotor 33 is rotatably mounted by means of a bearing element strip 39 formed in the mouthpiece 3a, which is shown in Figure 5d. In use, as the rotor 33 rotates, the end of the rotor moves back and forth along the bearing element strip 39.

The mouthpiece 3a is shown in detail in Figure 5d. A pathway for gaseous flow is defined from the lumen of the stator housing 34 around the bearing element strip 39, and through the mouthpiece 3a.

In use, when the smoker draws on the smoking article 1, the cavities 36 formed between the rotor 33 and the internal surface 35 of the stator housing 34 are drawn towards the mouthpiece 3a of the vibration component 32, causing the rotor 33 to rotate. The centre of mass of the rotor 33 does not lie on the axis of rotation and therefore rotation of the rotor 33 causes vibration of the vibration component 32.

In the embodiment shown in Figure 5, the rotor 33 is circular in cross-section, and the internal surface 35 of the stator housing 34 comprises a double lobed helix. In general, any progressive cavity arrangement may be used in which the internal surface 35 of the housing 34 comprises one lobe more than the rotor 33. For example, the vibration component may comprise a double lobed rotor located within a triple lobed cavity, or a triple lobed rotor located within a quadruple lobed cavity. In these embodiments in which the rotor comprises two or more lobes, the bearing element strip and elongated slot may be modified.

Numerous other variations and modifications are possible. For example, the rotor 33 may be eccentrically weighted to enhance the vibration generated by the vibration component 32. Imbalanced forces may be further tuned by alteration of the rotor diameter and/or pitch.

Embodiments of the type shown in Figure 5 are positive displacement vibration components and as a result, the lag time between the smoker commencing the draw and perceiving the resulting vibration is minimised.

Many modifications and variations of the vibration components 4, 18, 27, 32, are possible. For example, instead of a ball turbine 20, fan turbine 5, axial turbine 28, progressive cavity turbine 33, or cylindrical turbine, other types of turbine, having various different shapes, could be employed. However, in embodiments a spherical shape may advantageously be employed to optimise the volume of flow which is used to drive the turbine, especially when employed in combination with a substantially cylindrical conduit, so as to optimise interaction between the smoke and the turbine.

Still further, although the ball 20, fan 5, screw element 28, and rotor 33, of Figures 1 to 5 are described as being flow-driven (driven by a flow of smoke), alternatively, driving means such as a motor, or mechanical means, may be provided to rotate the ball 20, fan 5, screw element 28, rotor 33, or other suitable rotary member. The motor may be activated by means of a switch, or the motor may be activated by flow drawn by the user.

The vibration components shown in Figures 1 to 5 comprise a rotary member arranged to rotate in response to receiving flow. In these and other embodiments, vibration may be produced as a result of the rotary members being arranged so that the centre of mass of the rotary member does not lie on the axis of rotation. This may be achieved using one or a combination of different approaches including: the addition to the rotary member of an unbalancing mass; the use of a rotary member in which the vanes or blades do not have uniform shapes, sizes, densities, and/or masses; and/or the use of an eccentrically positioned axis of rotation. In some embodiments, simple inherent variations in the mass distribution resulting from the manufacturing process may be sufficient to provide vibration as the rotary member rotates.

In other embodiments, other vibration components may be used which do not comprise rotary members. These embodiments do not form part of the claimed invention. Figure 6a illustrates another vibration component 40 in place between a tobacco rod 2 and a filter plug 3. As shown, component 40 comprises a cylindrical body 41 having an inner surface 42 defining a smoke conduit 43 which encloses an element in the form of a movable member 44. Movable member 44 is thus positioned within the path of smoke along the smoke conduit 43. The movable member 44 is adapted so that smoke flow drives movement of the movable member 44, causing it to repeatedly impact the inner surface 42 of smoke conduit 43 so as to cause vibration.

Figure 6b is a perspective view of the vibration component 40, with part of the body removed. As shown, one end of body 41 includes a bar 45 extending perpendicular to the longitudinal axis of the component 40. Bar 45 defines two openings 46 at the end of the body to permit the passage of smoke into the component 40 from the tobacco rod 2. Body 41 also includes an opening 47 at the opposing end of the body to permit passage of smoke out of component 40 to the filter 3.

As shown, the movable member 44 comprises a tear-drop-shaped element which is cantilevered on a support in the form of a spring 48, so as to be movable relative to body 41. Instead of a spring, another suitable support could be used, formed for example from a suitable flexible, resilient material.

As shown, the support 48 is anchored to the bar 45. The member 44 is formed of a suitably light material so that it is readily moved about the smoke/air flow. Thus, in use, smoke is drawn from the tobacco rod 2, through the openings 46 and into the smoke conduit 43, where it jostles the movable member 44 about. The member 44 repeatedly impacts the inner surface 42 of body 41 and in this way causes vibration of the component 40. The vibration is imparted to the tobacco rod 2 and filter 3 and is perceived tactually by the smoker. The number of impacts per second depends on the smoke flux, such that vibration increases and decreases according to the amount of draw on the smoking article.

In addition, or as an alternative, to impaction of the inner surface 42, movement of the movable member 44 may be transmitted to the body 41 via the bar 45 thereby causing vibration of the vibration component 40.

As shown in the example of Figure 6a, movable member 44 moves in a constricted region 49 of the conduit 43. The constricted region provides a venturi effect which increases the velocity of the smoke flow in this region.

As shown in Figures 6a-6c, the body 41 may also include air inlets 51a, 51b, 51c formed in its periphery. As shown, inlets 51a direct air directly to the filter 3. Inlets 51b direct air into the smoke conduit 43. Inlets 51c direct air to the movable member 44. In some embodiments, air flow drawn from the inlets 51c is sufficient to cause movement of the member 44. This may be in addition to forces resulting from the smoke flow, or alternatively, in some embodiments air flow may move the member 44 instead of smoke flow.

The inner surface 42 of body 41 may have a coefficient of restitution of ≈ 0.75 to 1. The movable member 44 may have substantially the same coefficient of restitution as the walls.

According to embodiments, the movable member 44 comprises an aerodynamic element adapted to vortex shed, flutter, or otherwise create a dynamic flow instability. In this way, the smoke/air flow may drive continual motion of the movable member. Parameters of the support 48 and movable member (e.g: the masses of the movable member 44 and/or the stiffness) and elasticity of the spring 48) may be selected to obtain a resonant system, e.g: a simple harmonic resonant system.

In some implementations, the movable member suspended within the flow sheds vortexes on opposite sides of its surface at a certain velocity of smoke flow. These vortexes may shed at predictable frequency, proportional to the speed of the flow. As a consequence of the shedding of each vortex, there is a corresponding pressure change, resulting in a lift force effect acting perpendicular to the flow of the fluid. Because the vortex shedding occurs on opposing sides of the member 44 in alternate sequence, the corresponding force is therefore approximately sinusoidal, imparting a regular perturbation force. The frequency of the perturbation force can be matched with the resonant frequency of the movable member and support, creating an enhanced resonance.

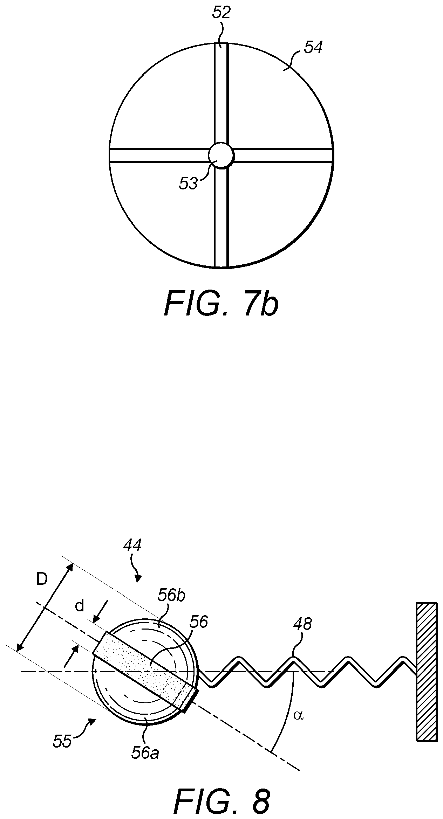

Figure 7a illustrates a variation of the vibration component 40 of Figure 6a, and the same reference numerals are retained for corresponding features. As shown, in the example of Figure 7a, the movable member 44 comprises a ball, suspended by a resilient support (e.g: spring) 48. As shown in Figure 7b, which shows an end view, one of the end faces of the vibration component of Figure 7a is provided with spokes 52, which extend radially from a hub 53 and which define openings 54 to permit the passage of smoke into the component 40. As shown in Figure 7a, one end of the resilient support 48 is anchored to the hub 53.

Figure 8 shows a further variation in which the movable member comprises a ball 55 having a surface adapted to provide enhanced movement in the flow of smoke and air. As shown, the ball 55 has a raised ridge 56 which runs circumferentially around its diameter. The ridge 56 divides the ball into two halves 56a, 56b. One half 56a has a smooth (e.g: polished) surface and the other half 56b has a rougher, more uneven, surface. As a result, opposing sides of the ball have different aerodynamic properties. As shown, the ball 55 is movably tethered so that the ridge 56 makes an angle α (e.g: 45°) with the support 48. The differing surface textures on opposing sides may give rise to enhanced movement. In some implementations, enhanced movement may result from the different surface textures causing uneven and turbulent air flow, causing vortices to shed away at varying positions around the surface area of the ball. As the ball is jostled about it gives rise to further instability, and the process is continued and reinforced. In some embodiments, the nature and periodicity of the vortex shedding may vary depending on temperature, humidity and velocity of the smoke flow.

Many further variations of the vibration component 40 are possible. For example, although the movable member 44 is described above as repeatedly impacting the inner surface of the cavity to cause vibration, alternatively, or in addition, in some embodiments, vibration may be caused in a different way. For example, movement of the member 44 may cause movement of the support and this may cause vibration the body 41. In some examples where vibration is transferred via support 48, the moving member 44 may not impact the inner surface of the cavity.

In an alternative variation, the movable member 44 may be loose (ie: the support 48 may be omitted) so that the smoke flow jostles the ball about freely in the cavity to repeatedly impact the inner surface of the cavity and cause vibration.

Furthermore, although the tear-drop-shaped element of Figure 6a is described above as cantilevered on a support, in some examples the support is made from a soft, floppy material, e.g: a suitable flexible plastic, to loosely tether the movable member 44. Further, rather than a tear drop shape, or a spherical ball, the moving element may have another suitable shape.

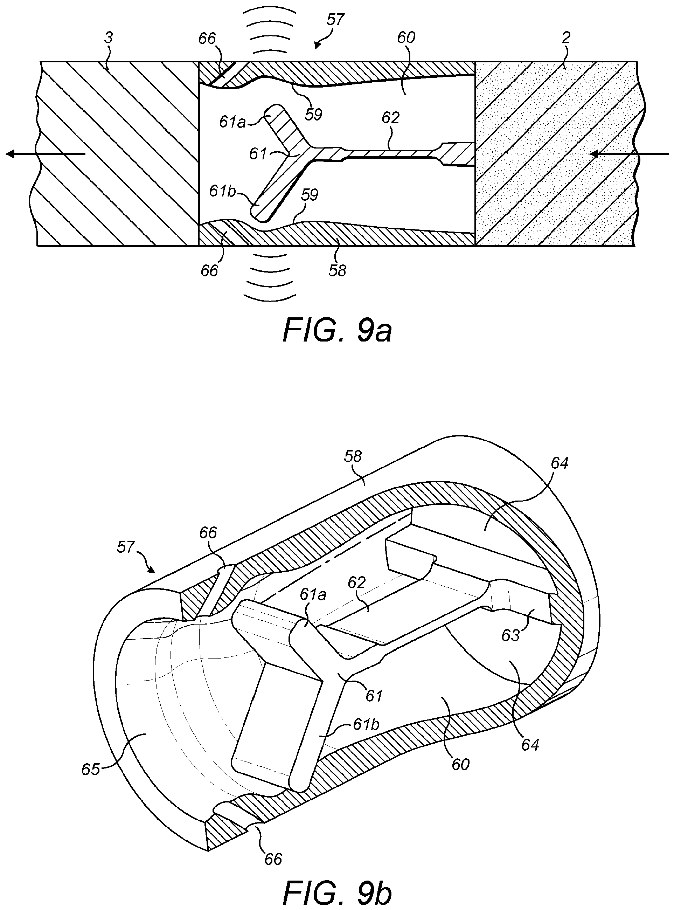

Figure 9a shows another vibration component 57 in place between a tobacco rod 2 and a filter plug 3. As shown, component 57 includes a cylindrical body 58 having an inner surface 59 defining a smoke conduit 60 which encloses a movable member comprising a shaped element 61 and a flexible tether 62, which holds the shaped element 61 in the smoke flow. As shown in Figure 9b one end of body 58 includes a bar 63 extending perpendicular to the longitudinal axis of the component 57. Bar 63 defines two openings 64 at the end of the body 58 to permit the passage of smoke into the component 57. Body 58 also includes an opening 65 at the opposing end of the body 58 to permit passage of smoke out of component 57.

Element 61 and tether 62 may be integral with one another and formed for example from a soft plastic material which allows the movable member to flutter in the passing flow. As shown in Figure 9a and 9b, shaped element 61 comprises two opposing members 61a, 61b, which are each arranged at an angle to the smoke flow direction. As shown, one of the members 61b is longer than the other member 61a so that the members 61a, 61b are arranged asymmetrically with respect to one another.

The movable member 61, 62 is unstable and tends to move in the flow of smoke, so that the element 61 repeatedly collides against the inner surface 59 of the body 58 and causes vibration.

The average number of collisions per second is higher when the smoke flux is higher, such that the vibration increases and decreases according to the amount of draw on the smoking article 1. The outer edges of shaped element 61 and/or the inner surface 59 of body 58 may be made from or covered with a material of high coefficient of restitution, so as to enhance vibration.

As is also described above with reference to Figures 6a-6c, in some implementations the movable member 61, 62 sheds vortexes at a certain velocity of smoke flow, which imparts a perturbation force. It is thought that the phenomenon of vortex shedding is also responsible for the simulated "swimming" motion of known fishing lures as they are drawn through water, as described for example in US2002/0194770, US2005/0193620 and US2009/0126255. In some embodiments, the shaped element 61 and tether 62 of the vibration component 57 may be formed from a similar material to such fishing lures, e.g: a suitable flexible, soft plastic material, and may be similarly shaped, so that the movable member 61, 62 "swims" in the flow of smoke.

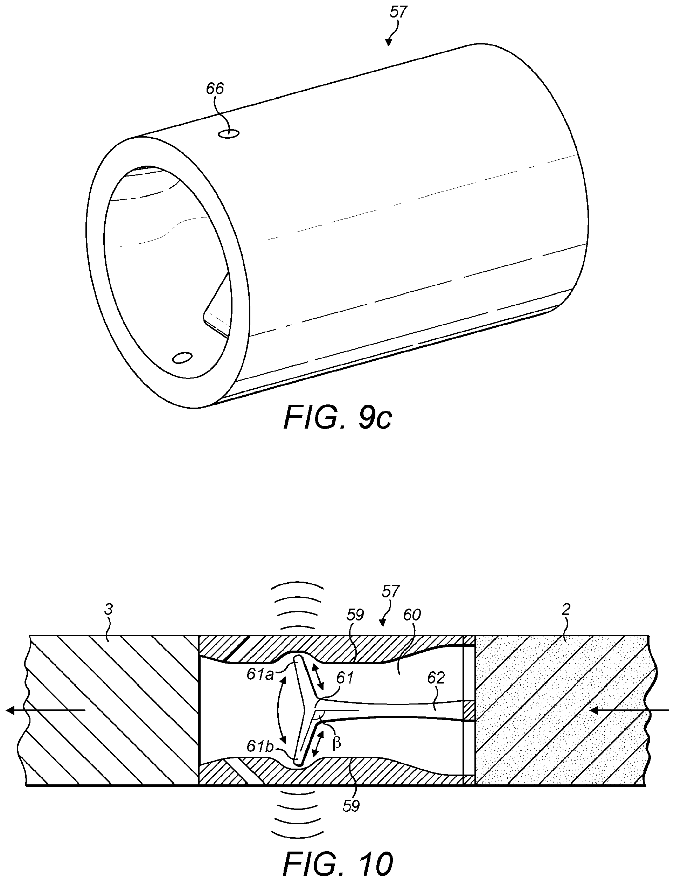

As shown in Figures 9a and 9b, and in Figure 9c, which shows an external view of the component 57, the body 58 may include air inlets 66 to permit diluting air to be drawn in and mixed with the smoke flow. In some embodiments, some or all of the diluting air may be drawn via one or more chambers comprising, for example, a flavourant.

Those skilled in the art will appreciate that the element 61 may be of any suitable symmetric or asymmetric shape so as to flutter in the flow of smoke drawn through the conduit 60. The shape of the inner surface 59 of the body may be varied depending on the shape of the element 61.

Figure 10 illustrates a variation of the component 57 of Figure 9a, and the same reference numerals are used to indicate corresponding features. As shown, in the variation of Figure 10, the opposing members 61a, 61b are arranged symmetrically. As shown, the members are each arranged at an angle β to the smoke flow direction, where β is greater than 90° and may for example be 110 °.

Many variations and modifications are possible. For example, in some embodiments, the tether 62 may be hingedly connected at the bar 63. In some examples, the element 61 may pull longitudinally on the bar 63 and in this way impart vibration to the body of the vibration component. In other examples, the element 61 may be loose (ie: untethered) so as to flutter freely within the conduit 60, causing vibration by repeatedly impacting the inner surface 59.

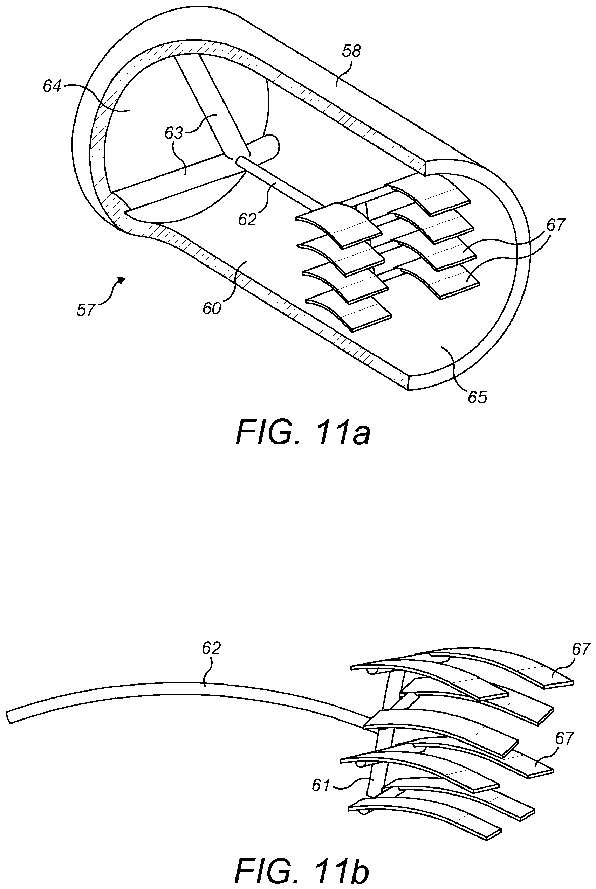

Figure 11a shows a further modification of the vibration component 57 shown in Figures 7a and 8, and the same reference numerals are used for corresponding features. In the embodiment of Figure 11a, the shaped element 61 is configured to flutter in response to the smoke that is drawn through the vibration component 57. This fluttering causes movement of the vibration component 57.

As shown in Figure 11b, the shaped element 61 comprises a number of aerofoils 67. The aerofoils 67 are shaped and positioned for significant interaction with the smoke that is drawn through the smoke conduit 60. The flexible tether 62, the shaped element 61, the bars 63, and the aerofoils 67 are formed of a suitably light and flexible material which may be readily moved about in the smoke flow. Thus, in use, smoke is drawn from the tobacco rod 2, through the opening 65 and into the vibration component 57. As it is drawn through the smoke conduit 60, the smoke passes over and around the aerofoils 67. The aerodynamic forces exerted by the smoke on the aerofoils 67 create lift which causes the tether 62 to increasingly bend. Bending of the tether 62 changes the angle at which the smoke flow contacts the aerofoils 67. Eventually, an angle is reached at which aerofoils 67 are incapable of creating lift and therefore the shaped element 61 springs back to its starting position before the process is repeated. In this way, the shaped element 61 is driven to flutter by drawn flow. This movement of the shaped element 61 and tether 62 is transmitted via the bars 63 to the cylindrical body 58. Thus, the vibration is perceived tactually by the smoker. The degree of movement of the shaped element 61 may depend on the smoke flux, such that vibration increases and decreases according to the amount of draw on the smoking article.

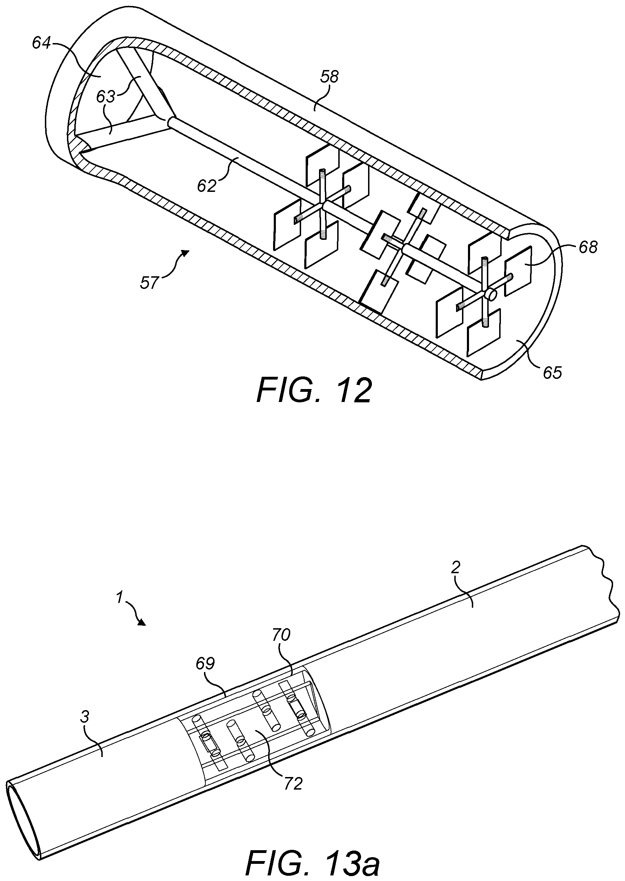

A variation of the embodiment shown in Figure 11a is shown in Figure 12, and the same reference numerals are used for corresponding features. In the embodiment of Figure 12 the shaped element 61 comprises a plurality of bluff bodies 68. Each bluff body 68 of the shaped element 61 is configured to provide resistance to the smoke flow. The number, shape, and position of the bluff bodies 68 may be varied to optimise the desired effect.

Thus, in use, smoke drawn through the vibration component 57 is resisted by the bluff bodies 68 of the shaped element 61. The action of the smoke flow on the bluff bodies 68 induces torsional forces in the flexible tether 62 which causes it to flutter and twist. This movement causes vibration of the vibration component 57 which is perceived tactually by the smoker. The degree of movement of the shaped element 61 may be dependent on the smoke flux.

To enhance vibration of any of the vibration components of the types shown in Figures 6a to 12, in some examples the component 57 may include two or three or four shaped elements 61. An end region of the body 58 may include multiple bars 63. A different element 61 may be tethered to each bar 63.

Many different vibration components may be produced which are caused to vibrate as a result of the resistance to the smoke flow and consequent vortex shedding offered by one or a sequence of bluff bodies.

For example, in embodiments the von Kármán vortex street effect may be harnessed to induce vibration of the vibration component. Figure 13a illustrates a smoking article 1 comprising a vibration component 69 in place between a tobacco rod 2 and a filter plug 3. As shown, the vibration component 69 comprises a cylindrical body 70 having an inner surface 71 defining a smoke conduit which encloses an element in the form of a movable member 72. The movable member 72 is thus positioned within the path of gaseous flow along the smoke conduit.

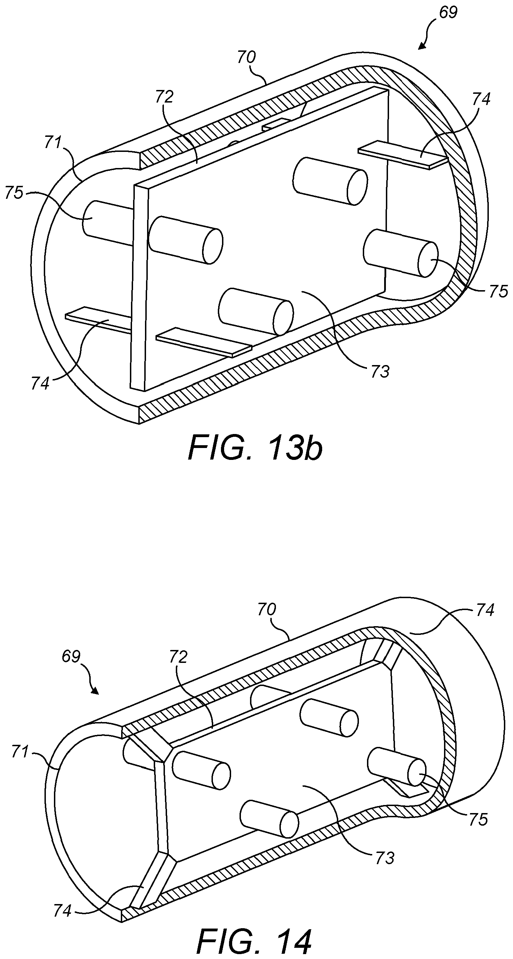

As shown in more detail in Figure 13b, the movable member 72 comprises an inflexible, substantially flat planar base element 73. The base element 73 is attached to the inner surface 71 of the body 70 by means of one or more supporting elements in the form of thin cantilevered beams 74. The movable member 72 also comprises a number of inflexible cylindrical bluff bodies 75 which are attached substantially perpendicularly to at least one face of the base element 73.

The cantilevered beams 74 are formed of a suitably flexible material so that the movable member 72 is readily moved about within the smoke conduit by the gaseous flow. Thus, in use, smoke is drawn from the tobacco rod 2 and into the smoke conduit, where vortexes are induced in the gaseous flow as it is drawn around the cylindrical bluff bodies 75. The interaction of the vortices created by the various bodies 75 causes movement of the movable member 72. This movement excites the cantilevered beams 74 causing vibration of the component 69.

Parameters of the movable member 72 and cantilevered beams 74, including the elasticity of the movable member 72 and/or cantilevered beams 74, and the size, shape and position of the bluff bodies 75 may be selected to obtain a resonant system. For example, in some implementations, the bluff bodies 75 may shed vortexes on opposite sides of their surface at a certain velocity of smoke flow. As a consequence of the shedding of each vortex, there is a corresponding pressure change, resulting in a lift force effect which may cause the movable member 72 to move. The vortexes may shed at a predictable frequency, proportional to the speed of the flow, generating a repeating pattern of swirling vortexes within the gaseous flow. The bluff bodies 75 may be positioned to amplify the magnitude of the vortexes and thus increase the movement of the movable member 72. Because the vortex shedding may occur in a repeating pattern, the frequency of the perturbation forces can be matched with the resonant frequency of the movable member 72 and cantilevered beams 74, creating an enhanced resonance.

Figure 14 illustrates a variation of the vibration component of Figure 13a, and the same reference numerals are retained for corresponding features. As shown in Figure 14, the movable member 72 is elastically attached to the inner surface 71 of the body 70, in this case by means of a plurality of elastic support members 74. In use, vortexes are induced in the gaseous flow as it is drawn around the cylindrical bluff bodies 75 causing movement of the movable member 72. Movement of the movable member 72 is transmitted via the elastic supports 74 and causes vibration of the component 69.

In embodiments of the type shown in Figure 13a and 14, the extent of the movement of the movable member 72 depends on the smoke flux, such that vibration of the vibration component 69 increases and decreases according to the amount of draw on the smoking article 1.

The vibration components shown in Figures 4a to 14 comprise a movable member 44, 55, 61, 72 comprising an aerodynamic element 44, 55, 61, 67, 68, 72 arranged to move in response to receiving flow. The moveable member 44, 55, 61, 72 is arranged to move (for example flutter) without any preset pattern in response to receiving flow.

Still further, although the moveable members of any of the embodiments described above are described as being flow-driven (ie: driven by a flow), in alternative embodiments, driving means such as a motor, which may be an electric or piezoelectric motor, or mechanical means, may be provided to move the moveable member. The motor may be activated by means of a switch, or the motor may be activated by flow drawn by the user.

Many further variations of the vibration components described above are possible. For example, although vibration components are generally shown in the figures positioned between a tobacco rod 2 and a filter rod 3, alternatively, a vibration component may be formed within a filter rod, for example positioned between two filter rod components. In some embodiments, the vibration component may be positioned at the extreme mouth end of the smoking article, for example, adjacent to the filter rod component.

Exemplary vibration components according to examples of the invention may for example be formed from a suitable biodegradable material such as polyhydroxyalkanoates (PHA), polylactides (PLA), polyvinyl alcohols (PVOH), or starch-based materials. Other materials such as polyethylene (PE), polyamides, polyether ether ketones (PEEK), polyurethanes (PU), polyoxymethylene (POM), cellulose-based materials, or other suitable materials may also be used. In some embodiments, the vibration components may include carbon.

According to various embodiments of the invention, exemplary vibration components may generate a vibration frequency of between 3 Hz and 1000 Hz. In embodiments, a vibration frequency in the range 50 Hz to 150 Hz, such as between 60 Hz and 100 Hz, or approximately 70 Hz or approximately 80 Hz may be generated. In embodiments, a pulse frequency in the range 30 Hz to 1000 Hz, such as between 50 Hz and 200 Hz, or such as between 60 Hz and 70Hz, may be generated. In some examples, the vibration may result in a displacement of ± 0.1 mm.

According to embodiments, a smoking article may be provided with multiple vibration components of the same or different types, to enhance vibration. In embodiments, vibration components may be longitudinally arranged in a smoking article.

Although examples described herein relate to a smoking article comprising a vibration apparatus in the form of a vibration component, the various vibration apparatuses of this disclosure could alternatively be provided as part of another flow delivery article, e.g: a flow delivery article for delivering an aerosol other than smoke.

## Claims

1. A flow delivery article to deliver a gaseous flow to the mouth of a user, comprising:
a flow pathway; and
a flow-driven vibration component (4, 18, 27, 32) configured to provide vibration driven by flow passing along the flow pathway, thereby to provide tactile stimulation to the user, and wherein the vibration component comprises a rotary member (5, 20, 28, 33) arranged to rotate in response to receiving flow.

2. A flow delivery article to deliver a gaseous flow to the mouth of a user, wherein the flow delivery article comprises:
a source of inhalable agent; and
a vibration component (4, 18, 27, 32) to provide tactile stimulation to the user, wherein the vibration component is longitudinally adjacent to or longitudinally spaced from the source of inhalable agent, and wherein the vibration component comprises a rotary member (5, 20, 28, 33) arranged to rotate in response to receiving flow.

3. A flow delivery article as claimed in claim 2, wherein the vibration component is located at the mouth end of the flow delivery article.

4. A flow delivery article as claimed in claim 2 or claim 3, comprising a smoking article having a filter (3), wherein the filter is arranged between the vibration component and the source of inhalable agent.

5. A flow delivery article as claimed in claim 2, comprising a smoking article (1) having a filter (3), wherein the vibration component is arranged within the filter.

6. A flow delivery article as claimed in claim 2, comprising a smoking article (1) having a filter (3), wherein the vibration component is arranged between the filter and the source of inhalable agent.

7. A flow delivery article as claimed in any preceding claim, wherein vibration of the vibration component increases when the flow drawn by the user increases and decreases when the flow drawn by the user decreases.

8. A flow delivery article as claimed in any preceding claim, wherein the flow delivery article has a peripheral region to contact the lips of the user, and wherein the vibration component is arranged to vibrate said peripheral region to provide tactile stimulation to the user's lips.

9. A flow delivery article as claimed in any preceding claim, wherein the flow delivery article has a peripheral region to contact the fingers of the user, and wherein the vibration component is arranged to vibrate said peripheral region to provide tactile stimulation to the user's fingers.

10. A flow delivery article as claimed in any preceding claim, comprising an eccentrically-weighted rotary member (5, 20, 28, 33).

11. A flow delivery article as claimed in any of claims 1-9, comprising an eccentrically-mounted rotary member (5, 20, 33).

12. A flow delivery article as claimed in any preceding claim, wherein the vibration component comprises a flow conduit (26, 35) arranged to preferentially direct flow towards a peripheral surface of the rotary member.

13. A flow delivery article as claimed in any preceding claim, wherein the rotary member comprises a turbine (5, 20, 28, 33).

14. A flow delivery article as claimed in claim 13, wherein the turbine comprises one of:
a substantially spherical turbine (20), and wherein the smoking article comprises a flow conduit (19a, 25) to preferentially direct flow towards one of the hemispheres (20a, 20b) of the spherical turbine; and
a fan turbine (5); and
a positive displacement turbine (28); and
an axial turbine (28); and
a progressive cavity turbine (33).

15. A flow delivery article as claimed in claim 14, wherein the progressive cavity turbine comprises a rotor (33) which is circular in cross-section and a stator housing (34) which comprises a double lobed helical lumen (35).

16. A flow delivery article as claimed in any preceding claim, wherein the vibration component includes a flow pathway therethrough, wherein the vibration component is configured to vibrate in response to receiving flow drawn through the flow pathway (24, 43), wherein the flow pathway includes a venturi section (24).

17. A flow delivery article as claimed in any preceding claim, comprising a smoking article (1).

18. A flow delivery article as claimed in claim 17, wherein the smoking article (1) comprises a tobacco rod component (2) and a filter rod component (3).

19. A flow delivery article as claimed in any of claims 2 to 6, comprising a motor configured to drive vibration of the vibration component.

20. A filter (3) for a smoking article (1) the filter (3) comprising a vibration component (4, 18, 27, 32) to provide tactile stimulation to a user, the vibration component (4, 18, 27, 32) comprising a rotary member (5, 20, 28, 33) arranged to rotate, in response to receiving a gaseous flow, in order to provide the tactile stimulation.

## Patentansprüche

1. Flussfreisetzungsartikel zur Abgabe einer gasförmigen Strömung an den Mund eines Benutzers, umfassend:
einen Strömungsweg; und
eine durch Strömung angetriebene Vibrationskomponente (4, 18, 27, 32), die zur Bereitstellung einer Vibration ausgelegt ist, die durch eine entlang des Strömungswegs fließende Strömung angetrieben wird, um dadurch taktile Stimulation für den Benutzer bereitzustellen, und wobei die Vibrationskomponente ein Drehelement (5, 20, 28, 33) umfasst, das ausgelegt ist, sich als Reaktion auf eine erhaltene Strömung zu drehen.

2. Flussfreisetzungsartikel zur Abgabe einer gasförmigen Strömung an den Mund eines Benutzers, wobei der Flussfreisetzungsartikel Folgendes umfasst:
eine Quelle eines inhalierbaren Mittels; und
eine Vibrationskomponente (4, 18, 27, 32) zur Bereitstellung taktiler Stimulation für den Benutzer, wobei die Vibrationskomponente längs neben oder längs im Abstand von der Quelle des inhalierbaren Mittels angeordnet ist, und wobei die Vibrationskomponente ein Drehelement (5, 20, 28, 33) umfasst, das ausgelegt ist, sich als Reaktion auf eine erhaltene Strömung zu drehen.

3. Flussfreisetzungsartikel nach Anspruch 2, wobei die Vibrationskomponente am Mundende des Flussfreisetzungsartikels angeordnet ist.

4. Flussfreisetzungsartikel nach Anspruch 2 oder Anspruch 3, umfassend einen Rauchartikel mit einem Filter (3), wobei der Filter zwischen der Vibrationskomponente und der Quelle des inhalierbaren Mittels angeordnet ist.

5. Flussfreisetzungsartikel nach Anspruch 2, umfassend einen Rauchartikel (1) mit einem Filter (3), wobei die Vibrationskomponente im Filter angeordnet ist.

6. Flussfreisetzungsartikel nach Anspruch 2, umfassend einen Rauchartikel (1) mit einem Filter (3), wobei die Vibrationskomponente zwischen dem Filter und der Quelle des inhalierbaren Mittels angeordnet ist.

7. Flussfreisetzungsartikel nach einem der vorhergehenden Ansprüche, wobei die Vibration der Vibrationskomponente zunimmt, wenn die vom Benutzer eingezogene Strömung zunimmt, und abnimmt, wenn die vom Benutzer eingezogene Strömung abnimmt.

8. Flussfreisetzungsartikel nach einem der vorhergehenden Ansprüche, wobei der Flussfreisetzungsartikel eine Umfangsregion für Kontakt mit den Lippen des Benutzers aufweist und wobei die Vibrationskomponente angeordnet ist, diese Umfangsregion zum Vibrieren zu bringen, um eine taktile Stimulation für die Lippen des Benutzers bereitzustellen.

9. Flussfreisetzungsartikel nach einem der vorhergehenden Ansprüche, wobei der Flussfreisetzungsartikel eine Umfangsregion für Kontakt mit den Fingern des Benutzers aufweist und wobei die Vibrationskomponente angeordnet ist, die Umfangsregion zum Vibrieren zu bringen, um eine taktile Stimulation für die Finger des Benutzers bereitzustellen.

10. Flussfreisetzungsartikel nach einem der vorhergehenden Ansprüche, umfassend ein exzentrisch gewichtetes Drehelement (5, 20, 28, 33).

11. Flussfreisetzungsartikel nach einem der Ansprüche 1-9, umfassend ein exzentrisch gewichtetes Drehelement (5, 20, 33).

12. Flussfreisetzungsartikel nach einem der vorhergehenden Ansprüche, wobei die Vibrationskomponente eine Durchflussleitung (26, 35) umfasst, die angeordnet ist, eine Strömung bevorzugt zu einer Umfangsfläche des Drehelements zu leiten.

13. Flussfreisetzungsartikel nach einem der vorhergehenden Ansprüche, wobei das Drehelement eine Turbine (5, 20, 28, 33) umfasst.

14. Flussfreisetzungsartikel nach Anspruch 13, wobei die Turbine eines der folgenden umfasst:
eine im Wesentlichen kugelförmige Turbine (20) und wobei der Rauchartikel eine Durchflussleitung (19a, 25) umfasst, um eine Strömung bevorzugt zu einer der Halbkugeln (20a, 20b) der kugelförmigen Turbine zu leiten; und
eine Fächerturbine (5); und
eine positive Verdrängerturbine (28); und
eine axiale Turbine (28); und
eine progressive Hohlraumturbine (33).

15. Flussfreisetzungsartikel nach Anspruch 14, wobei die progressive Hohlraumturbine einen Rotor (33) mit einem kreisförmigen Querschnitt und ein Statorgehäuse (34) mit einem doppelgelappten spiralförmigen Lumen (35) umfasst.

16. Flussfreisetzungsartikel nach einem der vorhergehenden Ansprüche, wobei die Vibrationskomponente einen Strömungsweg durch sie hindurch aufweist, wobei die Vibrationskomponente ausgelegt ist, als Reaktion auf eine erhaltene Strömung, die durch den Strömungsweg (24, 43) eingezogen wurde, zu vibrieren, wobei der Strömungsweg einen Venturi-Abschnitt (24) aufweist.

17. Flussfreisetzungsartikel nach einem der vorhergehenden Ansprüche, umfassend einen Rauchartikel (1) .

18. Flussfreisetzungsartikel nach Anspruch 17, wobei der Rauchartikel (1) eine Tabakstrangkomponente (2) und eine Filterspitzenkomponente (3) umfasst.

19. Flussfreisetzungsartikel nach einem der Ansprüche 2 bis 6, umfassend einen Motor, der zum Antreiben der Vibration der Vibrationskomponente ausgelegt ist.

20. Filter (3) für einen Rauchartikel (1), wobei der Filter (3) eine Vibrationskomponente (4, 18, 27, 32) zur Bereitstellung einer taktilen Stimulation für einen Benutzer umfasst, wobei die Vibrationskomponente (4, 18, 27, 32) ein Drehelement (5, 20, 28, 33) umfasst, das angeordnet ist, sich als Reaktion auf eine erhaltene gasförmige Strömung zu drehen, um die taktile Stimulation bereitzustellen.

## Revendications

1. Article émetteur de flux pour émettre un flux gazeux vers la bouche d'un utilisateur, comprenant:
un chemin de passage de flux; et
un composant à vibration provoquée par un flux (4, 18, 27, 32) configuré de manière à fournir une vibration provoquée par un flux passant le long du chemin de passage de flux, afin de procurer ainsi une stimulation tactile à l'utilisateur, et dans lequel le composant à vibration comprend un élément rotatif (5, 20, 28, 33) qui est agencé de manière à tourner en réponse à la réception du flux.

2. Article émetteur de flux pour émettre un flux gazeux vers la bouche d'un utilisateur, dans lequel l'article émetteur de flux comprend:
une source de agent inhalable; et
un composant à vibration (4, 18, 27, 32) pour procurer une stimulation tactile à l'utilisateur, dans lequel le composant à vibration est longitudinalement adjacent à ou longitudinalement espacé de la source d'agent inhalable, et dans lequel le composant à vibration comprend un élément rotatif (5, 20, 28, 33) qui est agencé de manière à tourner en réponse à la réception du flux.

3. Article émetteur de flux selon la revendication 2, dans lequel le composant à vibration est situé à l'extrémité côté bouche de l'article émetteur de flux.

4. Article émetteur de flux selon la revendication 2 ou la revendication 3, comprenant un article à fumer comprenant un filtre (3), dans lequel le filtre est agencé entre le composant à vibration et la source d'agent inhalable.

5. Article émetteur de flux selon la revendication 2, comprenant un article à fumer (1) comprenant un filtre (3), dans lequel le composant à vibration est agencé à l'intérieur du filtre.

6. Article émetteur de flux selon la revendication 2, comprenant un article à fumer (1) comprenant un filtre (3), dans lequel le composant à vibration est agencé entre le filtre et la source d'agent inhalable.

7. Article émetteur de flux selon l'une quelconque des revendications précédentes, dans lequel la vibration du composant à vibration augmente lorsque le flux aspiré par l'utilisateur augmente et diminue lorsque le flux aspiré par l'utilisateur diminue.

8. Article émetteur de flux selon l'une quelconque des revendications précédentes, dans lequel l'article émetteur de flux présente une région périphérique destinée à entrer en contact avec les lèvres de l'utilisateur, et dans lequel le composant à vibration est agencé de manière à faire vibrer ladite région périphérique afin de procurer une stimulation tactile aux lèvres de l'utilisateur.

9. Article émetteur de flux selon l'une quelconque des revendications précédentes, dans lequel l'article émetteur de flux présente une région périphérique destinée à entrer en contact avec les doigts de l'utilisateur, et dans lequel le composant à vibration est agencé de manière à faire vibrer ladite région périphérique afin de procurer une stimulation tactile aux doigts de l'utilisateur.

10. Article émetteur de flux selon l'une quelconque des revendications précédentes, comprenant un élément rotatif lesté de façon excentrique (5, 20, 28, 33).

11. Article émetteur de flux selon l'une quelconque des revendications 1 à 9, comprenant un élément rotatif monté de façon excentrique (5, 20, 33).

12. Article émetteur de flux selon l'une quelconque des revendications précédentes, dans lequel le composant à vibration comprend un conduit de flux (26, 35) agencé de manière à diriger de façon préférentielle le flux en direction d'une surface périphérique de l'élément rotatif.

13. Article émetteur de flux selon l'une quelconque des revendications précédentes, dans lequel l'élément rotatif comprend une turbine (5, 20, 28, 33).

14. Article émetteur de flux selon la revendication 13, dans lequel la turbine comprend un élément parmi:
une turbine (20) sensiblement sphérique, et dans lequel l'article à fumer comprend un conduit de flux (19a, 25) pour diriger de façon préférentielle le flux en direction de l'une des hémisphères (20a, 20b) de la turbine sphérique; et
une turbine à ventilateur (5); et
une turbine à déplacement positif (28); et
une turbine axiale (28); et
une turbine à cavité progressive (33).

15. Article émetteur de flux selon la revendication 14, dans lequel la turbine à cavité progressive (33) comprend un rotor (33) qui présente une section transversale circulaire et un carter de stator (34) qui présente une lumière hélicoïdale à double lobe (35).

16. Article émetteur de flux selon l'une quelconque des revendications précédentes, dans lequel le composant à vibration inclut un chemin de passage de flux à travers celui-ci, dans lequel le composant à vibration est configuré de manière à vibrer en réponse à la réception d'un flux aspiré à travers le chemin de passage de flux (24, 43), dans lequel le chemin de passage de flux comprend une section de venturi (24).

17. Article émetteur de flux selon l'une quelconque des revendications précédentes, comprenant un article à fumer (1).

18. Article émetteur de flux selon la revendication 17, dans lequel l'article à fumer (1) comprend un composant de tige de tabac (2) et un composant de tige de filtre (3).

19. Article émetteur de flux selon l'une quelconque des revendications 2 à 6, comprenant un moteur configuré de manière à provoquer la vibration du composant à vibration.

20. Filtre (3) pour un article à fumer (1), le filtre (3) comprenant un composant à vibration (4, 18, 27, 32) destiné à procurer une stimulation tactile à un utilisateur, le composant à vibration (4, 18, 27, 32) comprenant un élément rotatif (5, 20, 28, 33) agencé de manière à tourner, en réponse à la réception d'un écoulement de gaz, dans le but de procurer la stimulation tactile.
